(19) Europäisches Patentamt
European Patent Office
Office européen des brevets

(11) Publication number: **0 035 862**
**B1**

(12) # EUROPEAN PATENT SPECIFICATION

(45) Date of publication of patent specification: **13.06.84**

(21) Application number: **81300879.4**

(22) Date of filing: **03.03.81**

(51) Int. Cl.[3]: **C 07 D 219/10,**
C 07 C 59/105,
C 07 H 7/02, C 07 H 11/04,
A 61 K 31/435,
A 61 K 31/70

(54) **Pharmaceutically acceptable salts of 4'-(9-acridinylamino) methanesulfon-m-anisidide, their production, and pharmaceutical compositions containing such salts.**

(30) Priority: **11.03.80 US 129503**

(43) Date of publication of application:
**16.09.81 Bulletin 81/37**

(45) Publication of the grant of the patent:
**13.06.84 Bulletin 84/24**

(84) Designated Contracting States:
**AT BE CH DE FR GB IT LI LU NL SE**

(56) References cited:
**EP - A - 0 025 705**
**US - A - 1 674 923**

**JOURNAL OF MEDICINAL CHEMISTRY, vol. 19, no. 12, published in december 1976 Washington, US B.F. CAIN et al.: "Potential antitumor agents. 20", pages 1409-16**

(73) Proprietor: **WARNER-LAMBERT COMPANY**
**201 Tabor Road**
**Morris Plains New Jersey 07950 (US)**

(72) Inventor: **Fisher, James Royal**
**2329 N. Washington**
**Royal Oak Michigan 48073 (US)**
Inventor: **Kulier, Charles Peter**
**1181 Oak Hampton Road**
**Holland Michigan 49423 (US)**

(74) Representative: **Jones, Michael Raymond et al,**
**HASELTINE LAKE & CO. 28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 035 862 B1**

## Description

This invention relates to pharmaceutically acceptable salts of 4'-(9-acridinylamino)-methanesulfon-*m*-anisidide, to processes for their production, and to a pharmaceutical composition including such salts; such salts and composition are useful as antineoplastic agents.

The compound 4'-(9-acridinylamino)-methanesulfon-*m*-anisidide (hereinafter abbreviated to *m*-AMSA) has the following formula:—

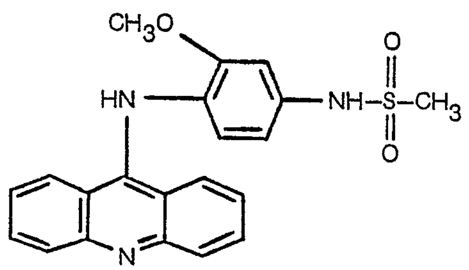

In view of the following published national applications relating to the gluconic acid salt of *m*-AMSA, the present applicants have voluntarily limited the scope of the claims for Belgium, France, Germany (Fed. Rep.), Luxembourg, Netherlands, Sweden and United Kingdom, and submitted separate claims for those states:—

BE—A—887220
FR—A—2474493
DE—A—3102026
LU—A—83081
NL—A—8100292
SE—A—8100341
GB—A—2068729.

According to one aspect of the present invention, there is provided a compound having the following general formula:—

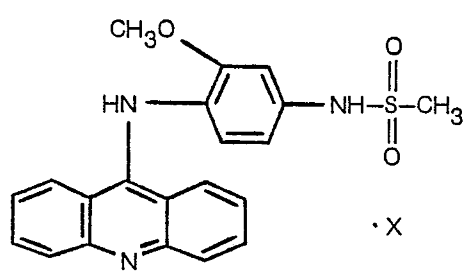

wherein X is acid having a total of from three to six carbon atoms including the carbon atom in any carboxylic group present, two to five hydroxy groups and only one acidic group selected from —$CO_2H$ and —$OPO_3H_2$ with the proviso that, when the total number of carbon atoms in the acid is three or four, the acidic group is —$OPO_3H_2$.

The compounds of the present invention are salts having a relatively high degree of solubility in water and capable of remaining in solution for a sufficient period of time to permit the solution to be introduced into a mammal. The term "solution" is used in this specification to mean a material which is in a liquid state capable of completely or almost completely passing through 0.22 $\mu$ millipore filter. Certain of the salts of the present invention do form true solutions; however, this may not always be the case.

The present invention thus provides pharmaceutically acceptable acid-addition salts of the formula

*m*—AMSA·X

wherein X is as defined above.

The acid X of the formula *m*—AMSA·X, may be in the D,L or a racemic form. In addition, the salt *m*—AMSA·X may exist in a solvated form. All of the foregoing forms are intended to be encompassed by the formula *m*—AMSA·X.

A pharmacetutical composition comprising *m*—AMSA·X and a pharmaceutically acceptable carrier may be sterile, lyophilized and contained in a hermetically sealed container.

The preferred acids represented by X are D-gluconic acid, D-glucuronic acid, D-galacturonic acid and β-glycerophosphoric acid which give rise to 4′-(9-acridinylamino)methanesulfon-*m*-anisidide D-gluconate, 4′-(9-acridinylamino)methanesulfon-*m*-anisidide D-glucuronate, 4′-(9-acridinylamino)-methanesulfon-*m*-anisidide D-galacturonate, and 4′-(9-acridinylamino)-methanesulfon-*m*-anisidide β-glycerophosphate, respectively.

The present invention also relates to methods for preparing said salts, and pharmaceutical compositions utilizing said salts. The salts and compositions can be used for treating mammals having certain viral or microbial infections, cancer or a tumor, the salts preferably being in the form of the disclosed pharmaceutical compositions.

The compound 4′-(9-acridinylamino)methanesulfon-*m*-anisidide, generally referred to as *m*-AMSA, has been found to have a high degree of activity in treating mammals in studies using mice:

"The Experimental Antitumour Properties of Three Congeners of the Acridylmethanesulphonanilide (AMSA) Series", B. F. Cain and G. J. Atwell, Europe. J. Cancer, Vol. 10, pp. 539—549. Pergamon Press (1974);

"Potential Antitumor Agents. 16. 4′-(Acridin-9-ylamino)methanesulfonanilides", Bruce F. Cain, Graham J. Atwell and William A. Denny, Journal of Medicinal Chemistry, Vol. 18, pp. 1110—1117 (1975);

"Structure-Activity Relationships for Thiolytic Cleavage Rates of Antitumor Drugs in the 4′-(9-Acridinylamino)methanesulfonanilide Series", Bruce F. Cain, Willian Robert Wilson and Bruce C. Baguley, Molecular Pharmacology, Vol. 12. pp. 1027—1035 (1976);

"Potential Antitumor Agents. 17. 9-Anilino-10-methylacridinium Salts", Bruce F. Cain. Graham J. Atwell and Willian A. Denny, Journal of Medicinal Chemistry, Vol. 19, pp 772—777 (1976);

"Potential Antitumor Agents. 26. Anionic Congeners of the 9-Anilinoacridines", Willian A. Denny, Graham J. Atwell and Bruce F. Cain, Journal of Medicinal Chemistry. Vol. 21. pp. 5—10 (1978).

The compound *m*-AMSA is generally prepared by coupling 9-chloroacridine to 4′-amino-methanesulfon-*m*-anisidide under acid-catalyzed conditions which is described in the following references:

"Potential Antitumor Agents. 12. 9-Anilinoacridines", G. J. Atwell, B. F. Cain and R. N. Seelye, Journal of Medicinal Chemistry, Vol. 15, pp. 611—615 (1972);

"The Experimental Antitumor Properties of Three Congeners of the Acridylmethanesulphonanilide (AMSA) Series", B. F. Cain and G. J. Atwell, Europ. J. Cancer, Vol. 10, pp. 539—549. Pergamon Press (1974);

"Potential Antitumor Agents. 16. 4′-(Acridin-9-ylamino)methanesulfonanilides", Bruce F. Cain, Graham J. Atwell and William A. Denny, Journal of Medicinal Chemistry, Vol. 18, pp. 1110—1117 (1975);

"Potential Antitumor Agents. 17. 9-Anilino-10-methylacridinium Salts", Bruce F, Cain, Graham J. Atwell and William A. Denny, Journal of Medicinal Chemistry, Vol. 19, pp. 772—777 (1976);

"Potential Antitumor Agents. 20. Structure-Activity-Site Relationships for the 4′-(9-Acridinylamino)alkanesulfonanilides", Bruce F. Cain and Graham J. Atwell, Journal of Medicinal Chemistry, Vol. 19, pp. 1409—1416 (1976);

"Potential Antitumor Agents. 23. 4′-(9-Acridinylamino) alkanesulfonanilide Congeners Bearing Hydrophilic Functionality", Bruce F. Cain, Graham J. Atwell and William A. Denny, Journal of Medicinal Chemistry, Vol. 20, pp. 987—996 (1977).

Unfortunately, *m*-AMSA is a compound which is not water soluble. It also has been found that the salts of *m*-AMSA, such as the hydrochloride salt, are also of a low order of solubility in water (see Table I). The journal of Medicinal Chemistry, 1976, Vol 19, No. 12, pages 1409—16 discloses the methane sulphonic acid salt of *m*-AMSA, whis is six times as water-soluble as the aforementioned hydrochloride salt, but even this increased solubility is far lower than desired for practical purposes, e.g. parenteral administration. If the compound is to be administered parenterally, a solution containing the compound must be prepared. At present, solutions employing dimethylacetamide as the solvent have been tried; however, the toxicity associated with this solvent makes this approach very undesirable. In addition, the dimethylacetamide is a potent solvent which presents problems as relates to the tubing and plastic syringes used when administering or storing the solution.

It has been found that certain acids when reacted with *m*-AMSA give salts having high degrees of water solubility. Depending upon the salt, concentration and temperature, standing may result in certain of the solutions of salts forming gels. However, a salt need only remain in solution for a period long enough for it to be administered to a patient. The stability of aqueous solutions of certain preferred compounds is given in Table II.

TABLE I

| Acid | Relative Solubility of *m*-AMSA Salts a,b<br>Rel. Solubility as Comp. to $H_2O$ |
|---|---|
| Galacturonic | 6133 |
| Glucose-6-Phosphoric | 3785 |
| Gluconic | 2815 |
| Ascorbic | 1213 |
| β-Glycerophosphoric | 1085 |
| Glucuronic | 923 |
| Lactic | 890 |
| Glyceric | 555 |
| Citric | 438 |
| Acetic | 234 |
| Propionic | 225 |
| Gallic | 225 |
| Isethionic | 213 |
| Malonic | 192 |
| D,L-Malic | 165 |
| Succinic | 154 |
| *d*-Tartaric | 138 |
| Glutaric | 102 |
| Mucic | 33 |
| Phosphoric | 29 |
| Salicylic | 18 |
| Glycolic | 18 |
| Benzoic | 10 |
| Methane-Sulfonic | 7.5 |
| Gentisic | 7.5 |
| Sulfuric | 6 |
| Nitric | 3 |
| Sulfamic | 2.8 |
| Hydrochloric | 1.2 |
| Water | 1 |

a) After solution is achieved, certain solutions upon standing set up like gels. The fact that on standing gels form in certain instances is not reflected in the table.

b) The values were obtained by adding *m*-AMSA to 0.1 M aqueous solutions, except gallic acid (0.05 M), mucic acid (0.01 M), salicylic acid (0.015 M), benzoic acid (0.015 M) and gentisic (0.03 M).

Although ascorbic acid, referred to in Table I above, can exist in the δ-lactone of 2,4,5,6-tetrahydroxy-3-ketohexanoic acid, especially when it exists as crystalline material, it nonetheless exists in its ketohexanoic acid form in solution, and thus is embraced by the present invention as a suitable acid X.

TABLE II
Stability of Preferred *m*-AMSA Salts

| *m*-AMSA Salt | *m*-AMSA Concentration mg/ml | Temperature °C | Results |
|---|---|---|---|
| gluconate | 5.2 | room temperature | a) after four days — fluid and clear to tyndall light.<br>b) after three weeks — fluid and clear to ordinary light, haze by tyndall light. |
| gluconate | 5.2 | 5° | a) after four days — solution gelled. |
| gluconate | 7.4 | room temperature | a) after three days — fluid and clear to tyndall light.<br>b) after three weeks — fluid and clear to ordinary light, haze by tyndall light. |
| gluconate | 7.4 | 5° | a) after three days — solution gelled. |
| gluconate | 10 | room temperature | a) after 18 hours — fluid and clear to tyndall light.<br>b) after 18 days — clear to ordinary light, haze by tyndall light. |
| gluconate | 10 | 5° | a) after 18 hours — solution gelled.<br>b) after 18 days — solution gelled* with slight flocculant precipitate. |

* all foregoing gluconate solutions of lower concentration that gelled at 5°C could be returned to a fluid state by warming to room temperature and shaking, at 10 mg/ml this could not be achieved after storing at 5°C.

TABLE II (Continued)
Stability of Preferred *m*-AMSA Salts

| *m*-AMSA Salt | *m*-AMSA Concentration mg/ml | Temperature °C | Results |
|---|---|---|---|
| glucuronate | 4.97 | room temperature | a) after 18 days — hazy to tyndall light and some particles. |
| glucuronate | 4.97 | 5° | a) after 18 days — solution gelled but liquifies on warming to room temperature with stirring (haze to tyndall light and slight precipitate). |
| β-glycerophosphate | 7.5 | room temperature | a) after three days — fluid and clear to tyndall light.<br>b) after three weeks — solution gelled but became fluid on shaking (haze to tyndall light). |
| β-glycerophosphate | 7.5 | 5° | a) after three days — gelled (warming to room temperature and shaking gave solution clear to tyndall light).<br>b) after three weeks — gelled (warming to room temperature and shaking gave solution hazy to tyndall light). |

It is also noted that solubility is enhanced by the presence of an excess of an acid of the formula X′ wherein X′ is an acid having a total of from three to six carbon atoms including the carbon atom in any carboxylic group present, two to five hydroxy groups and only one acidic group which may be a —$CO_2H$ group or a —$OPO_3H_2$ group with the proviso that when the total number of carbon atoms is three or four, the acidic group is $OPO_3H_2$.

The acid X′ may or may not be the same as the acid used in forming the salt of *m*-AMSA. Generally, the same acid is employed to form the salt of *m*-AMSA and supply the excess acid, since it is more convenient to use this approach to prepare the *m*-AMSA salt with excess acid (X and X′ are the same in this instance). The quantity of excess acid used, when compared to the *m*-AMSA salt on a molar basis, is about 0.2 moles to about 10.0 moles per mole of *m*-AMSA salt, preferably approximately 1.0 moles to approximately 6.0 moles.

The salts are prepared from *m*-AMSA base and the acid in an inert solvent. Although up to 10% molar excess of either reagent may be employed, equimolar proportions of *m*-AMSA base and the acid are preferred.

The *m*-AMSA base and the acid are dissolved in dimethylformamide, dimethylacetamide, methanol, anhydrous ethanol, 95% ethanol, isopropanol or combinations of the solvents at 35° to 100°C. After filtration of the solution, the salt is precipitated by addition of another solvent such as anhydrous ethanol, isopropanol, anhydrous diethyl ether or ethyl acetate and/or chilling at 5° to −20°C for 2 to 24 hours. The preferred method is to dissolve the *m*-AMSA base and acid in anhydrous ethanol and dimethylformamide at 70—100°, in boiling anhydrous ethanol or in boiling methanol. The solution is filtered and the salt separated from the filtrate by the following procedures. The dimethylformamide-anhydrous ethanol solution (about 70°C) is diluted with anhydrous ethanol; the anhydrous ethanol and the methanol solutions at 30—35°C are diluted with diethyl ether. The product is further precipitated by chilling the filtrates at 5° to −20°C for 16—24 hours. After collecting the product it is dried under reduced pressure at 50—60°C.

The compounds of the present invention are useful in treating certain viral infections, namely those caused by Avian myeloblastoma or vaccinia virus or certain bacterial infections, namely those caused by Salmonella typhimurium bacteria.

In addition, compounds of the present invention are useful antineoplastic agents in the treatment of mammals, such as dogs, cats, etc. Typical of the disease states wherein the compounds of this invention may be employed are leukemia, breast cancer, cellular hepatoma, melanoma, etc. The preferred route of administration is intravenous and the dose generally employed is from about 20 mg to about 500 mg/m² of body surface per day for one to five days preferably 30 mg to 100 mg/m² per day for about three days. The procedure may be repeated about every three weeks.

The compounds of the present invetion may also be administered orally in the form of capsules, tablets, syrups, etc., or rectally in the form of suppositories. Generally, higher doses are employed when using these routes, especially orally. Thus, when employing these routes about 20 mg to about 1.0 g/m² of body surface per day for from one to five days, preferably 50 mg to 800 mg/m² per day for about three days is used.

The compounds of the invention, when administered intravenously, are generally dissolved in water, sterifiltered, lyophilized and redissolved at the time of use. Preferably, an excess of acid is present to further improve solubility and minimize the chances of any blockage of the pores during sterifiltration step. An excess of an acid is less desirable if the sterilization process does not utilize a microfiltration step. The lyophilized material is dissolved in a non-toxic aqueous vehicle prior to administration. The vehicle may contain buffers, materials, to make the resultant solution isotonic, preservatives, etc., which are compatable with the compounds of this invention. Certain ions, such as halide ion, cause precipitation of the hydrohalide salt of *m*-AMSA and should be avoided.

In addition, the compounds of the present invention are orally administered, for example, with an inert diluent or with an assimilable edible carrier, or they may be compressed into tablets, or they may be incorporated directly with the food of the diet. For oral therapeutic administration, the active compounds of this invention may be incorporated with excipients and used in the form of tablets, troches, capsules, elixirs, suspensions, syrups and the like. Such compositions and preparations should contain at least 0.1% of active compound. The percentage in the compositions and preparations may, of course, be varied and may conveniently be between about 5% to about 75% or more of the weight of the unit. The amount of active compound in such therapeutically useful compositions or preparations is such that a suitable dosage will be obtained. Preferred compositions or preparations according to the present invention are prepared so that an oral dosage unit form contains between about 10 and 200 milligrams of active compound.

The tablets, troches, pills, capsules and the like may also contain the following: a binder such as gum tragacanth, acacia, corn starch or gelatin; an excipient such as dicalcium phosphate, a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. Various other materials may be present as coatings or to otherwise modify the physical form of the dosage unit, for instance, tablets, pills or capsules may be coated with shellac, sugar or both. A syrup or elixir may contain the active

compounds, sucrose as a sweetening agent, methyl and propyl parabens as preservatives, a dye, and a flavoring such as cherry or orange flavor. Of course, any material used in preparing any dosage unit form should be pharmaceutically pure and substantially non-toxic in the amounts employed.

Lastly, the compounds of this invention may be administered in the form of a suppository using glycerin, cocoa butter, etc., as a vehicle. In addition to the compounds of the invention, preservatives and/or coloring agents may also be incorporated into the suppository. The enhanced solubility of the *m*-AMSA salts may also give the certain advantages when administered by these routes.

The compounds of this invention may be administered in combination with other antineoplastic agents. The antineoplastics may be derived from the numerous classes of agents; such as: antibiotic derivatives, doxorubicin, mitomycin or actinomycin; antiestrogen agents, such as tamoxifen; antimetabolites, such as fluorouracil, carmustine, lomustine, methotrexate, mercaptopurine or thioguanine; cytotoxicagents, such as bleomycin, cyclophosphamide, busulfan, procarbazine or hyroxyurea; hormones, such as dromostanolone, ethinyl estradiol, megestrol, prednisone, methyltestosterone, chlorotrianisene or testolactone; nitrogen mustard derivatives, such as melphalan, chlorambucil, methlorethamine or TESPA; steroids, such as betamethasone or prednisolone, and other miscellaneous agents, such as vinblastine, vincristine, asparaginase, mitotane or cisplatin. When using combinations of two or more neoplastic agents, the dosage of *m*-AMSA salt may be reduced. The dosage ranges generally employed for the above cited antineoplastics which may be used with *m*-AMSA are known to the medical profession, see Physicians' Desk Reference, 34th Edition, Medical Economics Co. 1980.

The invention is illustrated by the following examples.

### Example 1

*m*-AMSA D—Gluconate

A solution of 3.93 gm of *m*-AMSA and 5.15 gm of D-gluconic acid-water solution (38% w/w) in 305 ml of specially denatured 3A anhydrous ethanol at 70—80°C is filtered with suction. The filtrate is cooled to 30—35°C and 100 ml of anhydrous diethyl ether is added with stirring. After chilling to 0—5°C with stirring, the mixture is stored about 16 hours at 5°C. The product is collected, washed with specially denatured 3A anhydrous ethanol and anhydrous diethyl ether (3:1) at 5°C, then with several portions of anhydrous ether and dried under reduced pressure at 50—55°C to give 3.95 gm of *m*-AMSA D-gluconate. The salt obtained by this process retains a small amount (about 1% of ethanol).

### Example 2

*m*-AMSA D—Galacturonate

A solution of 3.93 gm of *m*-AMSA and 2.12 gm of D-galacturonic acid monohydrate in 325 ml of specially denatured 3A anhydrous ethanol at 70—80°C is filtered with suction. The filtrate is stirred and chilled to 0—5°C, then stored at −20°C for about 16 hours. The product is collected, washed with cold (about −20°C) specially denatured 3A anhydrous ethanol, then with anhydrous diethyl ether and dried under reduced pressure at 50—55°C to give 3.9 gm of *m*-AMSA D-galacturonate. The salt obtained by this process retains a small amount about 3% of ethanol.

### Example 3

*m*-AMSA D—Glucose-6-phosphate

A mixture of 1.1804 gm of *m*-AMSA and 0.8576 gm of (91%) D-glucose-6-phosphoric acid monohydrate in 19.3 ml of dimethylformamide is heated on a hot plate until a complete solution is obtained at about 100°C. The hot, dark-colored solution is then diluted with an equal volume of warm (65—70°C) specially denatured 3A anhydrous ethanol and filtered immediately. Additional warm specially denatured 3A anhydrous ethanol (about 70°C) is used to rinse the funnel so that a total of 96.5 ml of ethanol is used. The mixture is allowed to cool to about 20—25°C with occasional swirling of the flask. The mixture is then cooled for several hours at 3—5°C and then for a similar period of time at −18 to −20°C. The solid is collected, washed thoroughly with cold (3—5°C) specially denatured 3A anhydrous ethanol, then with anhydrous diethyl ether and dried under reduced pressure at 39—40°C to give 1.38 gm of *m*-AMSA D-glucose-6-phosphate.

### Example 4

*m*-AMSA D—Glucuronate

A solution of 3.93 gm of *m*-AMSA and 2.0 gm of D-glucuronic acid in 325 ml specially denatured anhydrous ethanol at 70°—80°C is filtered with suction. After cooling to 30—35°C, 45 ml anhydrous diethyl ether is added with stirring and chilling to 5°C. After chilling about 16 hours at 5°C, the salt is collected, washed with cold (0—5°C) specially denatured 3A anhydrous ethanol, then with anhydrous diethyl ether and dried under reduced pressure at 50°C to give 4.5 gm of *m*-AMSA D-glucuronate.

Example 5

*m*-AMSA Gluconate Lyophilized Injectable Formulation

| | | per liter |
|---|---|---|
| 1. | *m*-AMSA Gluconate (11.77 mg/ml*) | 11.77 g |
| 2. | Gluconic Acid, 50% Solution | q.s. |
| 3. | Celite 521** | 1.5 g |
| 4. | Water for Injection, USP q.s. to make 1000 ml | |

The word "Celite" is a registered Trade Mark.

*Equivalent to 7.5 mg/ml of *m*-AMSA plus 3% intentional excess.

**If needed to clarify solution.

Method of Preparation (for 1000 ml):

A. Add sufficient 2 to approximately 900 ml of 4 in a suitable container to adjust the pH of the solution to approximately 2.2.

B. Add 1 slowly with continuous stirring until 1 completely dissolves.

C. Recheck pH and adjust to 2.3—2.7, if needed, with 2.

D. Add a sufficient amount of 4 to make 1000 ml of solution and mix well.

E. If necessary, clarify solution with 3 followed by suitable filtration to remove 3 (Whatman No. 1 filter paper, Millipore AW 19 membrane or equivalent).

F. Sterilize solution by filtration through a previously sterilized membrane (Millipore GS or equivalent) using appropriate prefiltration, if necessary.

G. Aseptically fill into previously sterilized vials (10 ml per vial).

H. Stopper vials loosely with rubber lyophilization stoppers and lyophilize in a suitable lyophilizer, (75 mg *m*-AMSA per vial).

I. At the conclusion of the lyophilization cycle, stopper and cap vials.

Example 6

*m*-AMSA Gluconate Injectable Formulation

| | per 1000 vials |
|---|---|
| *m*-AMSA gluconate (117.7 mg/vial*) | 117.7 g |

*Equivalent to 75 mg of *m*-AMSA + 3% intentional excess.

Method of Preparation:

1. Presterilize the *m*-AMSA gluconate.
2. Fill into appropriate** (10 ml—20 ml) previously sterilized vials.
3. Stopper and cap vials.

**If 10 ml vials are used 10.0 ml of Sterile Water for Injection is used to dissolve the sterile powder, the resulting concentraton will be equivalent to 7.5 mg/ml, *m*-AMSA, as the gluconate. If 20 ml vials are used and 20.0 ml of Sterile Water for Injection is used to dissolve the sterile powder, the resulting concentration will be 3.75 mg/ml.

Example 7

*m*-AMSA Gluconate Oral Formation

| | mg/cap | g/1000 caps |
|---|---|---|
| 1. *m*-AMSA Gluconate | 76.1* mg | 76.2 g |
| 2. Lactose USP Hydrous | 438.8 | 438.8 |
| 3. Polysorbate 80 USP | 5.0 | 5.0 |
| 4. Syloid 74 (silica gel) | 10.0 | 10.0 |
| 5. Alcohol SD 3A Anhydrous | 0.07 ml | 7.0 ml |
| | 530 mg | 530 g |

The words "polysorbate" and "Syloid" are registered Trade Marks.
*Equivalent to 50 mg *m*-AMSA Base.

Method of Preparation:

A. Dissolve 3 in 5 and added to 4 contained in a suitable blender. Blend thoroughly. Transfer the wet mass onto trays and dry at about 49°C (120°F) overnight. Add to 2 and mix well.

B. Screen 1 through a No. 60 screen, which is a 60 mesh screen with a sieve opening of 0.25 mm and a wire diameter of 0.162 mm. Add material from Step A and blend thoroughly. Pass through No. 60 screen (as above) and reblend.

C. Fill 530 mg of the powder mixture into No. "O" dark brown opaque hard gelatin capsules.

**Claims for the Contracting States: BE FR DE GB LU NL SE**

1. A compound having the following general formula:—

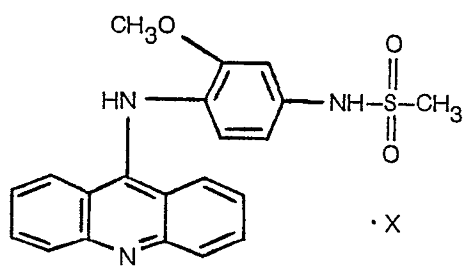

wherein X is an acid having a total of from three to six carbon atoms including the carbon atom of any carboxylic group present, two to five hydroxy groups and only one acidic group selected from —$CO_2H$ and —$OPO_3H_2$ with the proviso that, when the total number of carbon atoms in the acid is three or four, the acidic group is —$OPO_3H_2$, and with the proviso that X is not gluconic acid.

2. The compound of claim 1 having the name 4′-(9-acridinylamino)methanesulfon-*m*-anisidide D-glucuronate.

3. The compound of claim 1 having the name 4′-(9-acridinylamino)methanesulfon-*m*-anisidide D-galacturonate.

4. The compound of claim 1 having the name 4′-(9-acridinylamino)methanesulfon-*m*-anisidide β-glycerophosphate.

5. A pharmaceutical composition comprising a compound according to claim 1 and a pharmaceutically acceptable carrier therefor.

6. A pharmaceutical composition according to claim 5, which also includes an excess of an acid of formula X′ which is selected from the same class of acids as acid X.

7. A pharmaceutical composition according to claim 5 or 6, in which the composition is sterile, lyophilized and contained in a hermetically sealed container.

8. A pharmaceutical composition according to claim 5, 6 or 7, which also includes another anit-neoplastic agent.

9. A process for producing a compound according to claim 1, which process comprises reacting a compound having the following formula:—

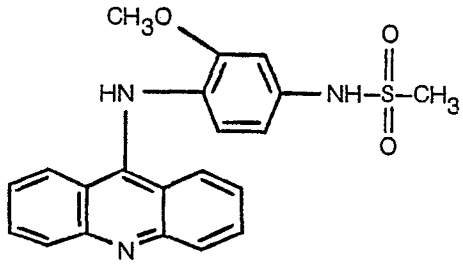

with an acid X wherein X is as defined in claim 1.

**Claims for the Contracting States: IT CH LI**

1. A compound having the following general formula:—

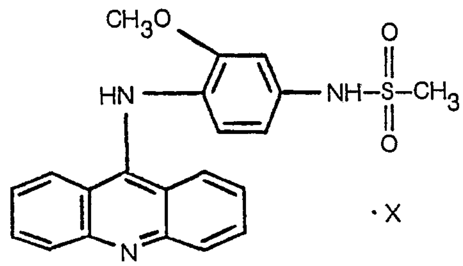

wherein X is an acid having a total of from three to six carbon atoms including the carbon atom of any carboxylic group present, two to five hydroxy groups and only one acidic group selected from —$CO_2H$ and —$OPO_3H_2$ with the proviso that, when the total number of carbon atoms in the acid is three or four, the acidic group is —$OPO_3H_2$.

2. The compound of claim 1 having the name 4′-(9-acridinylamino)methanesulfon-*m*-anisidide D-gluconate.

3. The compound of claim 1 having the name 4′-(9-acridinylamino)methanesulfon-*m*-anisidide D-glucuronate.

4. The compound of claim 1 having the name 4′-(9-acridinylamino)methanesulfon-*m*-anisidide D-galacturonate.

5. The compound of claim 1 having the name 4′-(9-acridinylamino)methanesulfon-*m*-anisidide β-glycerophosphate.

6. A pharmaceutical composition comprising a compound according to claim 1 and a pharmaceutically acceptable carrier therefor.

7. A pharmaceutical composition according to claim 6, which also includes an excess of an acid of formula X′ which is selected from the same class of acids as acid X.

8. A pharmaceutical composition according to claim 5, 6 or 7, which also includes another anti-lyophilized and contained in a hermetically sealed container.

9. A pharmaceutical composition according to claim 6, 7 or 8, which also includes another anti-neoplastic agent.

10. A process for producing a compound according to claim 1, which process comprises reacting a compound having the following formula:—

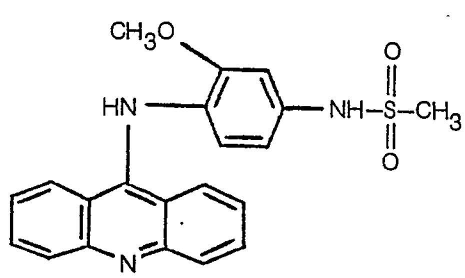

with an acid X wherein X is as defined in claim 1.

**Claims for the Contracting State: AT**

1. A process for producing a compound having the following general formula:—

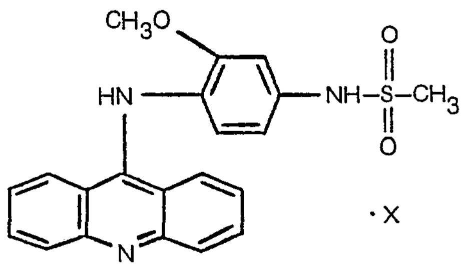

wherein X is an acid having a total of from three to six carbon atoms including the carbon atom of any carboxylic group present, two to five hydroxy groups and only one acidic group selected from —$CO_2H$ and —$OPO_3H_2$ with the proviso that, when the total number of carbon atoms is three or four, the acidic group is —$OPO_3H_2$; which process comprises reacting a compound of the following formula:—

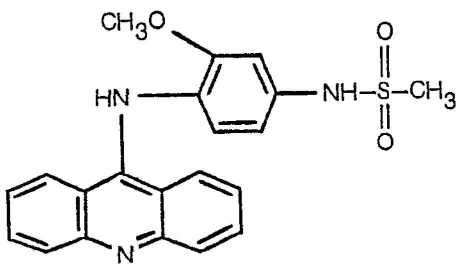

with an acid X wherein X is as defined above.

2. A process according to claim 1, wherein the acid X is D-gluconic acid.

3. A process according to claim 1, wherein the acid X is D-glucuronic acid.

4. A process according to claim 1, wherein the acid X is D-galacturonic acid.

5. A process according to claim 1, wherein the acid X is $\beta$-glycerophosphoric acid.

6. A process for producing a pharmaceutical composition which comprises admixing a compound having the formula I in claim 1 with a pharmaceutically acceptable carrier.

7. A process according to claim 6, which also includes sterilizing and lyophilizing the pharmaceutical composition.

8. A process according to claim 6 or 7, which also includes sealing the pharmaceutical composition in a hermetically sealed container.

9. A process according to claim 6, 7 or 8, which includes incorporating in the pharmaceutical composition an excess of an acid of formula X′ which is selected from the same class of acids as acid X.

10. A process according to any one of claims 6 to 9, which also includes incorporating in the pharmaceutical composition another anti-neoplastic agent.

**Revendications pour les Etats contractants: BE FR DE LU NL GB SE**

1. Un composé ayant la formule générale suivante:

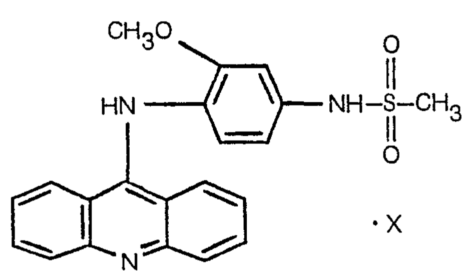

dans laquelle:
X est un acide ayant un total de 3 à 6 atomes de carbone, y compris l'atome de carbone de tout groupe

carboxylique présent, deux à cinq groupes hydroxy et seulement un groupe acide choisi parmi —$CO_2H$ et —$OPO_3H_2$ sous la réserve que, lorsque le nombre total d'atomes de carbone dans l'acide est égal à 3 ou à 4, le groupe acide est —$OPO_3H_2$, et sous la réserve que X ne soit pas l'acide gluconique.

2. Un composé selon la revendication 1, portant le nom 4'-(9-acridinylamino)méthanesulfone-*m*-anisilide-D-glucuronate.

3. Un composé selon la revendication 1, portant le nom de D-galacturonate de 4'-(9-acridinylamino)méthanesulfone-*m*-anisilide.

4. Un composé selon la revendication 1, portant le nom de beta-glycérophosphate de 4'-(9-acridinylamino)méthanesulfone-*m*-anisilide.

5. Une composition pharmaceutique comprenant un composé selon la revendication 1, et un véhicule pharmaceutiquement acceptable de celui-ci.

6. Une composition pharmaceutique selon la revendication 5, comprenant aussi un excès d'un acide de formule X' choisi dans la même classe d'acides que l'acide X.

7. Une composition pharmaceutique selon la revendication 5 ou 6, dans laquelle la composition est stérile, lyophilisée et contenue dans un conteneur scellé hermétiquement.

8. Une composition pharmaceutique selon la revendication 5, 6 ou 7, comprenant aussi un autre agent antinéoplasique.

9. Un procédé de préparation d'une composition selon la revendication 1, lequel procédé consiste à faire réagir une composé ayant la formule générale:

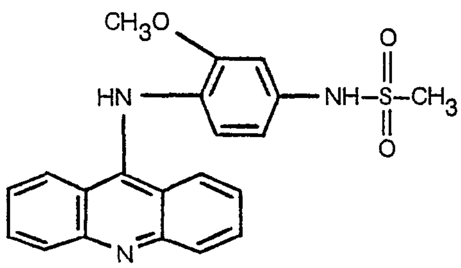

avec un acide X, dans laquelle X est tel que défini dans la revendication 1.

**Revendications pour les Etats contractants: IT CH LI**

1. Un composé ayant la formule générale suivante:

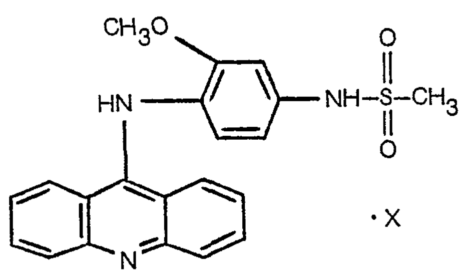

dans laquelle:
X est un acide ayant un total de 3 à 6 atomes de carbone y compris l'atome de carbone de tout groupe carboxylique présent, deux à cinq groupes hydroxy et seulement un groupe acide choisi parmi —$CO_2H$ et —$OPO_3H_2$ sous la réserve que, lorsque le nombre total d'atomes de carbone dans l'acide est égal à 3 ou à 4, le groupe acide est —$OPO_3H_2$.

2. Un composé selon la revendication 1, portant le nom 4'-(9-acridinylamino)méthanesulfone-*m*-anisilide-D-gluconate.

3. Un composé selon la revendication 1, portant le nom 4'-(9-acridinylamino)méthanesulfone-*m*-anisilide-D-glucuronate.

4. Un composé selon la revendication 1, portant le nom 4'-(9-acridinylamino)méthanesulfone-*m*-anisilide-D-galacturonate.

5. Un composé selon la revendication 1, portant le nom 4'-(9-acridinylamino)méthanesulfone-*m*-anisilide-beta-glycérophosphate.

6. Une composition pharmaceutique comprenant un composé selon la revendication 1, et un véhicule pharmaceutiquement acceptable pour celui-ci.

7. Une composition pharmaceutique selon la revendication 6, comprenant aussi un excès d'un acide de formule X' choisi dans la même classe d'acides que l'acide X.

8. Une composition pharmaceutique selon la revendication 6 ou 7, dans laquelle la composition est stérile, lyophilisée et est contenue dans un conteneur scellé hermétiquement.

9. Une composition pharmaceutique selon la revendication 6, 7 ou 8, qui comprend également un autre agent antinéoplasique.

10. Un procédé de préparation d'un composé selon la revendication 1, lequel procédé consiste à faire réagir un composé ayant la formule suivante:

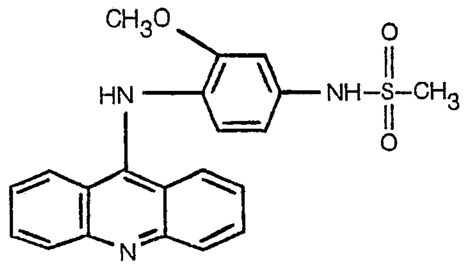

avec un acide X, dans lequel X est tel que défini dans la revendication 1.

**Revendications pour l'Etat contractant: AT**

1. Un procédé de préparation d'un composé ayant la formule générale suivante:

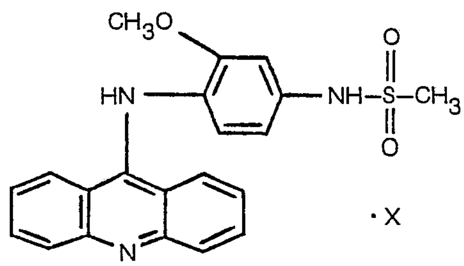

(I)

dans laquelle:
X est un acide possédant un total de 3 à 6 atomes de carbone, y compris l'atome de carbone de tout groupe carboxylique présent, de deux à cinq groupes hydroxy et seulement un groupe acide choisi parmi —$CO_2H$ et —$OPO_3H_2$ sous réserve que, lorsque le nombre total d'atomes de carbone est égal à 3 ou à 4, le groupe acide est —$OPO_3H_2$;
ce procédé étant caractérisé en ce que l'on fait réagir un composé de formule suivante:

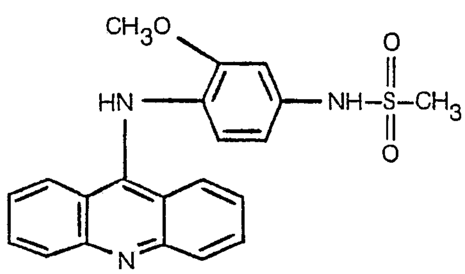

avec un acide X dans lequel X est tel que défini ci-dessus.

2. Un procédé selon la revendication 1, dans lequel l'acide X est l'acide D-gluconique.

3. Un procédé selon la revendication 1, dans lequel l'acide X est l'acide D-glucuronique.

4. Un procédé selon la revendication 1, dans lequel l'acide X est l'acide D-galacturonique.

5. Un procédé selon la revendication 1, dans lequel l'acide X est l'acide beta-glycérophosphorique.

6. Un procédé de production d'une composition pharmaceutique, caractérisé en ce que l'on mélange un composé ayant la formule I de la revendication 1 avec un véhicule pharmaceutiquement acceptable.

7. Un procédé selon la revendication 6, qui consiste aussi à stériliser et à lyophiliser la composition pharmaceutique.

8. Un procédé selon la revendication 6 ou 7, consistant aussi à sceller la composition pharmaceutique dans un conteneur scellé hermétiquement.

9. Un procédé selon la revendication 6, 7 ou 8, qui comprend l'incorporation dans la composition pharmaceutique d'un excès d'un acide de formule X' choisi dans la même classe d'acides que l'acide X.

10. Un procédé selon l'une quelconque des revendications 6 à 9, dans lequel on incorpore aussi dans la composition pharmaceutique un autre agent antinéoplasique.

**Patentansprüche für die Vertragsstaaten: BE FR DE LU NL GB SE**

1. Verbindung mit der folgenden allgemeinen Formel

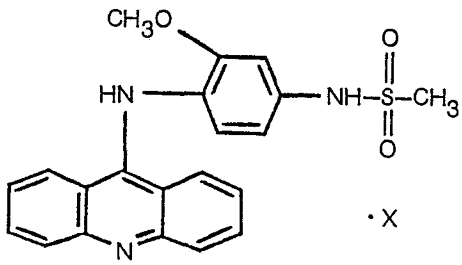

worin X eine Säure mit einer Gesamtzahl von drei bis sechs Kohlenstoffatomen, inklusive des Kohlenstoffatoms irgendeiner vorhandenen Carboxyl-Gruppe, zwei bis fünf Hydroxygruppen und nur eine saure Gruppe, ausgewählt aus $—CO_2H$ und $—OPO_3H_2$, bedeutet, mit der Maßgabe, daß, wenn die Gesamtzahl der Kohlenstoffatome in der Säure drei oder vier ist, die saure Gruppe $—OPO_3H_2$ ist, und mit der Maßgabe, daß X nicht Gluconsäure bedeutet.

2. Verbindung nach Anspruch 1 mit dem Namen 4'-(9-Acridinylamino)-methansulfon-m-anisidid-D-glucuronat.

3. Verbindung nach Anspruch 1 mit dem Namen 4'-(9-Acridinylamino)-methansulfon-m-anisidid-D-galacturonat.

4. Verbindung nach Anspruch 1 mit dem Namen 4'-(9-Acridinylamino)-methansulfon-m-anisidid-ß-glycerophosphat.

5. Pharmazeutische Zusammensetzung umfassend eine Verbindung nach Anspruch 1 und einen pharmazeutisch akzeptablen Träger für diese.

6. Pharmazeutische Zusammensetzung nach Anspruch 5, welche auch einen Überschuß eine Säure der Formel X' umfaßt, welche ausgewählt ist aus derselben Klasse von Säuren wie die Säure X.

7. Pharmazeutische Zusammensetzung nach Anspruch 5 oder 6, in welcher die Zusammensetzung steril, lyophilisiert und in einem hermetisch verschlossenen Behälter enthalten ist.

8. Pharmazeutische Zusammensetzung nach Anspruch 5, 6 oder 7, welche auch noch ein anderes anti-neoplastisches Mittel enthält.

9. Verfahren zur Herstellung einer Verbindung nach Anspruch 1, welches Verfahren gekennzeichnet ist durch die Umsetzung einer Verbindung der folgenden Formel

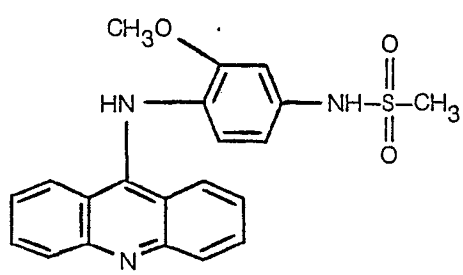

mit einer Säure X, worin X der in Anspruch 1 angeführten Definition entspricht.

**Patentansprüche für die Vertragsstaaten: IT CH LI**

1. Verbindung mit der folgenden allgemeinen Formel:

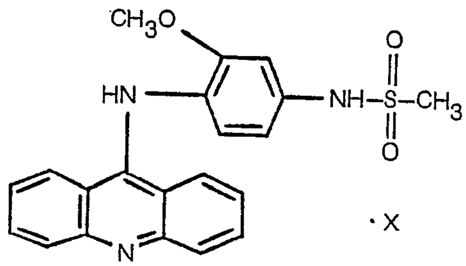

worin X eine Säure mit einer Gesamtzahl von drei bis sechs Kohlenstoffatomen, inklusive des Kohlenstoffatoms irgendeiner vorhandenen Carboxyl-Gruppe, zwei bis fünf Hydroxygruppen und nur eine saure Gruppe, ausgewählt aus $—CO_2H$ und $—OPO_3H_2$, bedeutet, mit der Maßgabe, daß, wenn die Gesamtzahl der Kohlenstoffatome in der Säure drei oder vier ist, die saure Gruppe $—OPO_3H_2$ ist.

2. Verbindung nach Anspruch 1 mit dem Namen 4'-(9-Acridinylamino)-methansulfon-m-anisidid-D-gluconat.

3. Verbindung nach Anspruch 1 mit dem Namen 4'-(9-Acridinylamino)-methansulfon-m-anisidid-D-glucuronat.

4. Verbindung nach Anspruch 1 mit dem Namen 4'-(9-Acridinylamino)-methansulfon-m-anisidid-D-galacturonat.

5. Verbindung nach Anspruch 1 mit dem Namen 4'-(9-Acridinylamino)-methansulfon-m-anisidid-ß-glycerophosphat.

6. Pharmazeutische Zusammensetzung umfassend eine Verbindung nach Anspruch 1 und einen pharmazeutisch akzeptablen Träger für diese.

7. Pharmazeutische Zusammensetzung nach Anspruch 6, welche auch einen Überschuß einer Säure der Formel X' umfaßt, welche ausgewählt ist aus derselben Klasse von Säuren wie die Säure X.

8. Pharmazeutische Zusammensetzung nach Anspruch 6 oder 7, in welcher die Zusammensetzung steril, lyophilisiert und in einem hermetisch verschlossenen Behälter enthalten ist.

9. Pharmazeutische Zusammensetzung nach Anspruch 6, 7 oder 8, welche auch noch ein anderes anti-neoplastisches Mittel enthält.

10. Verfahren zur Herstellung einer Verbindung nach Anspruch 1, welches Verfahren gekennzeichnet ist durch die Umsetzung einer Verbindung der folgenden Formel:

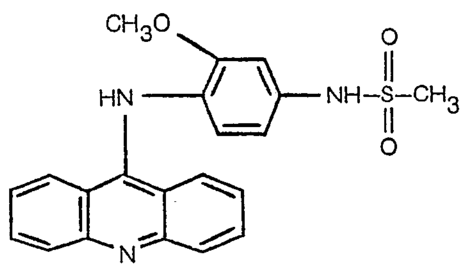

mit einer Säure X, worin X der in Anspruch 1 angeführten Definition entspricht.

**Patentansprüche für den Vertragsstaat: AT**

1. Verfahren zur Herstellung einer Verbindung mit der folgenden allgemeinen Formel:

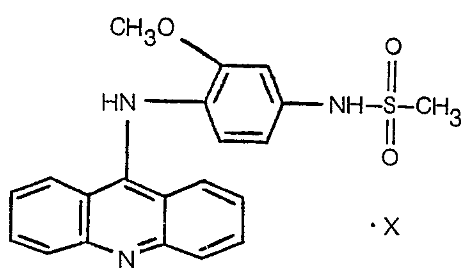

worin X eine Säure mit einer Gesamtzahl von drei bis sechs Kohlenstoffatomen, inklusive des Kohlenstoffatoms irgendeiner vorhandenen Carboxyl-Gruppe, zwei bis fünf Hydroxy-Gruppen und nur eine saure Gruppe, ausgewählt aus $-CO_2H$ und $-OPO_3H_2$, bedeutet, mit der Maßgabe, daß, wenn die Gesamtzehl der Kohlenstoffatome drei oder vier ist, die saure Gruppe $-OPO_3H_2$ ist; welches Verfahren dadurch gekennzeichnet ist, daß eine Verbindung der folgenden Formel

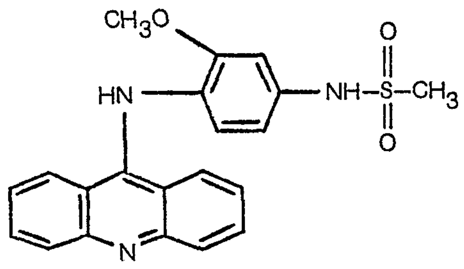

mit einer Säure X umgesetzt wird, wobei X der oben angegebenen Definition entspricht.

2. Verfahren nach Anspruch 1, dadurch gekennzeichnet, daß als Säure X D-Gluconsäure eingesetzt wird.

3. Verfahren nach Anspruch 1, dadurch gekennzeichnet, daß als Säure X D-Glucuronsäure eingesetzt wird.

4. Verfahren nach Anspruch 1, dadurch gekennzeichnet, daß als Säure X D-Galacturonsäure eingesetzt wird.

5. Verfahren nach Anspruch 1, dadurch gekennzeichnet, daß als Säure X ß-Glycerophosphorsäure eingesetzt wird.

6. Verfahren zur Herstellung einer pharmazeutischen Zusammensetzung, dadurch gekennzeichnet, daß eine Verbindung der Formel I nach Anspruch 1 mit einem pharmazeutisch akzeptablen Träger vermischt wird.

7. Verfahren nach Anspruch 6, dadurch gekennzeichnet, daß die pharmazeutische Zusammensetzung auch sterilisiert und lyophilisiert wird.

8. Verfahren nach Anspruch 6 oder 7, dadurch gekennzeichnet, daß die pharmazeutische Zusammensetzung weiters in einem hermetisch verschlossenen Behälter eingeschlossen wird.

9. Verfahren nach Anspruch 6, 7 oder 8, dadurch gekennzeichnet, daß in der pharmazeutischen Zusammensetzung ein Überschuß einer Säure der Formel X', welche aus derselben Klasse von Säuren wie die Säure X ausgewählt ist, inkorporiert wird.

10. Verfahren nach irgend einem der Ansprüche 6 bis 9, dadurch gekennzeichnet, daß in der pharmazeutischen Zusammensetzung auch noch ein weiteres anti-neoplastisches Mittel inkorporiert wird.